# EUROPÄISCHE PATENTSCHRIFT

(11) **EP 0 820 211 B1**
(45) Veröffentlichungstag und Bekanntmachung des Hinweises auf die Patenterteilung: **19.09.2001**
(21) Anmeldenummer: 96111047.5
(22) Anmeldetag: 09.07.1996
(51) Int. Cl.: H04R 25/00

(54) **Programmierbares Hörgerät**
Programmable hearing aid
Prothèse auditive programmable

(43) Veröffentlichungstag der Anmeldung: 21.01.1998
(73) Patentinhaber: Siemens Audiologische Technik GmbH, 91058 Erlangen (DE)
(72) Erfinder: Holube, Inga, Dr., 91052 Erlangen (DE); Sigwanz, Ullrich, Dipl.-Ing., 91058 Erlangen (DE); Martin, Raimund, Dipl.-Ing., 91330 Bammersdorf (DE); Zöls, Fred, Dipl.-Ing., 90592 Altentann (DE)
(74) Vertreter: Zedlitz, Peter, Dipl.-Inf.

(56) Entgegenhaltungen:
- EP-A- 0 415 677
- EP-A- 0 681 411
- DE-A- 4 234 964
- DE-A- 4 427 216
- GB-A- 2 055 020
- GB-A- 2 235 349
- US-A- 4 680 798
- US-A- 5 403 262
- HEARING INSTRUMENTS, Bd. 42, Nr. 9, 1.September 1991, Seiten 13-14, 16 - 17, XP000236829 AGNEW J: "ADVANCED DIGITAL SIGNAL PROCESSING SCHEMES FOR ITES"

## Beschreibung

Die Erfindung betrifft ein programmierbares digitales Hörgerät gemäß dem Oberbegriff des Patentanspruches 1.

Ein Hörgerät dieser Art ist aus der DE-B-27 16 336 bekannt. Bei diesem Hörgerät ist ein Mikrofon als Eingangssignalquelle vorgesehen, die an einen Verstärker angeschlossen ist, auf den ein Analog-Digital-Wandler folgt, der mit einem Rechnerblock verbunden ist, an dessen Ausgang ein Digital-Analog-Wandler liegt, der in einen Endverstärker mündet, dem als Ausgangswandler ein Hörer angeschlossen ist. Die Signalverarbeitungseinrichtung des programmierbaren Hörgerätes kann einen Mikroprozessor mit Speicher umfassen und als integrierter Baustein ausgeführt sein. Dabei können im Prozessor auch mehrere Eingangssignale, z.B. aus einem Mikrofon und einer Aufnahmeinduktionsspule, miteinander korreliert werden.

Als Tinnitus wird eine Krankheit beschrieben, bei der der Patient im Ohr oder Kopf Geräusche wahrnimmt, für die keine äußeren Ursachen vorhanden sind. Dies kann außerordentlich unangenehm sein und in schweren Fällen zu geistigen und körperlichen Störungen führen. In der wissenschaftlichen Literatur wird bereits seit vielen Jahren die Möglichkeit untersucht, das Tinnitus-Leiden dadurch zu lindern, daß man das TinnitusGeräusch mit einem dem Ohr zugeführten Schallsignal übertönt.

Aus der WO 90/07251 ist ein Tinnitus-Maskierungsgerät bekannt, mit einer in einem Gehäuse angeordneten elektronischen Schaltung und einem Hörer zur Erzeugung eines Schallspektrums, das den Tinnitus des Patienten überdecken kann, wobei zur Einstellung der Schallintensität ein Lautstärkeregler vorgesehen ist. Bei diesem Gerät ist die elektronische Schaltung so ausgebildet, daß das vom Hörer erzeugte Schallspektrum ein Linienspektrum mit einem Grundton enthält, wobei die Frequenz des Grundtons vom Benutzer einstellbar ist. Tinnitus-Maskierungsgeräte sind entweder eigenständige Geräte oder in Hörgeräte eingebaut, wobei man die Kombination aus einem Tinnitus-Masker und einem Hörgerät auch als Tinnitus-Instrument bezeichnet. Dieses Instrument wird beim Auftreten von Tinnitus und gleichzeitigem Hörverlust verwendet, was bei einem großen Anteil der Tinnitus-Patienten auftritt. Zur Behandlung von Tinnitus bzw. zur Tinnitus-Therapie arbeiten diese bekannten Geräte mit Analogschaltungen. Die Tinnitus-Masker generieren ein Rauschen, das im begrenzten Rahmen auf die Bedürfnisse hinsichtlich des Frequenz- und Pegelbereichs für jeden Tinnitus-Patienten individuell angepaßt werden kann. Ziel dieser Therapie ist es, das Maskierungsgeräusch so einzustellen, daß der Tinnitus durch den Patienten nicht mehr oder kaum noch wahrgenommen wird und statt dessen ein angenehmeres Maskierungsgeräusch hörbar ist.

Die US-A-4 680 798 beschreibt einen Tinnitusmasker, kombiniert mit einem Hörgerät, wobei das elektrische Signal des Tinnitusmaskers dem Hörgeräteverstärker zugeführt und als akustisches Signal zusammen mit dem Signal des Hörgeräts verwendet wird. Bei diesem Hörgerät mit analoger Signalverarbeitung führen die analogen Signalpfade über Verstärker zu einem Summierglied und zum Hörer. Auch die Signalanbindung eines Noise Generators ist analog und lediglich die Steuerung von einem Digital Controller über einen A/D Wandler zu switching gates ist digital, wobei ein analoges Audionutzsignal mit einem analogen Rauschsignal kombinierbar ist.

Die EP-A-0 415 677 betrifft ein Hörgerät mit einer Kompensation von Rückkopplungen, wobei zur Analyse der Rückkopplungen ein Rauschsignal erzeugt wird. Die dabei vorgesehene Signalgenerierung dient nur zur Analyse, nicht zur Darbietung für eine Tinnitustherapie.

Nachteilig bei den bekannten Tinnitus-Instrumenten ist, daß der Frequenzbereich und der Pegelbereich des Maskierungsrauschens nicht flexibel genug einstellbar ist. Außerdem hat die bekannte Art der Maskierung den Nachteil, daß sie ständig wirksam ist und daß das Gerät vom Patienten jeweils manuell abgeschaltet werden muß, falls er bei bestimmten Situationen normal hören möchte.

Aufgabe der Erfindung ist es, ein Hörgerät der eingangs genannten Art so auszubilden, daß es ohne die obengenannten Nachteile auch zur Tinnitus-Therapie sowie zur Retrainingstherapie von Tinnitus einsetzbar ist.

Erfindungsgemäß wird diese Aufgabe durch die Merkmale des Patentanspruches 1 gelöst. Vorteilhafte Ausgestaltungen der Erfindung sind in den Patentansprüchen 2 bis 11 gekennzeichnet.

Mit dem Hörgerät nach der Erfindung verbindet sich der Vorteil der Integration der Tinnitus-Therapie in ein digitales Hörgerät, womit eine hohe Flexibilität sowohl hinsichtlich des Frequenzbereiches als auch hinsichtlich des Pegels der Maskierungssignale erreicht wird. Darüber hinaus kann das Hörgerät sowohl als Tinnitus-Masker als auch als Tinnitus-Instrument oder nur als Hörgerät benutzt werden und verschiedene Programme des Hörgerätes können mit verschiedenen Funktionen belegt sein. Die programmierte Verarbeitung des Hörgerätes kann sehr flexibel durch den Austausch der Programmierdaten geändert werden, ohne daß die Hörgerätebauteile verändert werden müssen. Das erfindungsgemäße Hörgerät eignet sich sowohl zur klassischen Tinnitus-Therapie als auch zur Retrainingstherapie. Während die klassische Tinnitus-Therapie spektral gefärbte Signale mit mittleren bis hohen Pegeln benötigt, werden in der Retrainingstherapie wesentlich niedrigere Pegel und breitbandige Signale benutzt. Das Hörgerät nach der Erfindung ermöglicht eine Signalverarbeitung, vorgebbar durch verschiedene Parameter, so daß es unterschiedliche Programme sowohl als Hörgerät als auch als Tinnitus-Instrument umfaßt. Die Programmierbarkeit des Hörgerätes bietet die Möglichkeit einer Anpassung an den Hörschaden und/oder an die Tinnituskrankheit des Patienten und/oder an die jeweilige Hörsituation.

Durch den Einsatz eines programmierbaren Hörgerätesystems ist es möglich, nahezu alle gewünschten Signale zur Maskierung zu erzeugen. Die Beschränkung auf Breitbandrauschen entfällt. Dies ist vor allem bei normalhörenden Tinnitus-Patienten von Vorteil. Der Ton des Tinnitus kann genau bestimmt und die entsprechende Frequenz kann mit einem schmalbandigen Rauschen verdeckt werden. Bei der Auswahl des Signals besteht weiterhin die Möglichkeit, melodische Klangfolgen oder sonstige Töne zum Überdecken des Tinnitus zu generieren, wobei die Beschränkung auf Rauschen entfällt. Außerdem besteht die Möglichkeit, das Maskierungssignal bei akustischer Stimulation abzuschalten und erst bei längeren Ruhepausen zu aktivieren. Dies ermöglicht dem Träger des Hörgeräts, ohne Umschaltung von Maskierung auf Mikrofonbetrieb einem Gespräch zu folgen oder auf ein akustisches Signal zu reagieren. Tritt eine längere Ruhepause ein, so wird das Maskierungssignal langsam eingeblendet und überdeckt so das störende Tinnitusgeräusch.

Eine weitere Möglichkeit bildet die Erfindung darin, das Spektrum des akustischen Eingangssignals des Hörgerätes in Echtzeit zu analysieren und den Signalanteil des Frequenzbereichs, in dem der Tinnitus wirksam ist, auszuwerten. Ist im entsprechenden Frequenzbereich genügend Pegel vorhanden, so erfolgt keine Zumischung des Maskierungssignals. Bei Unterschreiten eines Schwellwertes wird das Maskierungssignal entsprechend zugemischt. Auf diese Weise kann ständig eine Anregung des entsprechenden Frequenzbereiches erreicht werden.

Die Maskierungsgeräusche werden im Signalprozessor des digitalen Hörgerätes erzeugt, gefiltert, verstärkt und zu dem Signalpfad des Hörgerätes hinzugemischt. Im Signalprozessor können sowohl Sinussignale als auch Schmalbandgeräusche unterschiedlicher Mittenfrequenz, Breitbandrauschen und Rauschen mit variabler Frequenzabhängigkeit erzeugt werden. Die Auswahl der Signale erfolgt beim Hörgeräteakustiker oder Arzt in Abstimmung mit dem Patienten. Dort wird auch die Filterung und die Verstärkung der Signale eingestellt. Damit ist das Hörgerät sowohl zur Maskierung des Tinnitus als auch zur Retrainingstherapie verwendbar. Da das Hörgerät mehrere Programmeinstellungen erlaubt, können diese mit verschiedenen Funktionen (Tinnitus-Masker, Tinnitus-Instrument oder Hörgerät) belegt werden. Dies ist vorteilhaft, da der Tinnitus zu verschiedenen Tageszeiten oder bei verschiedenen Tätigkeiten unterschiedlich stark sein kann und deshalb nicht immer ein Maskierungsgeräusch benötigt wird. Deshalb ist eine Auswahl zwischen einem Tinnitus-Masker oder Tinnitus-Instrument und einem Hörgerät von besonderer Bedeutung, da sie das Abschalten des Maskierungssignals erlaubt. Darüber hinaus kann das Hörgerät nach der Erfindung eine so hohe Klangqualität aufweisen, daß es auch von normalhörenden Tinnituspatienten ohne Beeinträchtigung der Klangqualität getragen werden kann. Wenn der Patient mit der Konfiguration des Gerätes nicht mehr zufrieden ist, dann kann vom Hörgeräteakustiker oder vom Arzt eine Neuanpassung durch andere, in den Speicher des Hörgerätes einlesbare Parameterwerte erfolgen.

Mit dem Hörgerät nach der Erfindung sind ferner die folgenden Ausführungen oder Weiterbildungen möglich:
- Der Pegel und/oder die Filterung des Maskierungssignals sind vom Patienten mit Hilfe von Bedienelementen am Hörgerät veränderbar.
- Das Hörgerät verstärkt den Frequenzbereich, der für die Beeinflussung des Tinnitus notwendig ist, mehr als aufgrund des Hörverlustes notwendig wäre, damit eine Maskierungswirkung ohne zusätzliche Maskierungssignale erreichbar ist. Dabei kann eine zusätzliche Dynamikkompression das Überschreiten der Unbehaglichkeitsschwelle vermeiden.
- Der Pegel der Maskierungssignale ist in Abhängigkeit vom Signalpegel veränderbar.
- Mehrere Maskierungssignale, die künstlich erzeugt oder in natürlichen Umgebungen aufgenommen werden, sind im Hörgerät speicherbar. Der Tinnitus-Patient kann im laufenden Hörgerätebetrieb zwischen diesen Signalen auswählen. Diese Signale können sowohl gefiltert als auch im Pegel verändert werden.

Weitere Einzelheiten und Vorteile der Erfindung werden nachfolgend anhand der in der Zeichnung dargestellten Ausführungsbeispiele erläutert.

Es zeigen:
Figur 1 ein Blockschaltbild eines programmierbaren, digitalen Hörgerätes,
Figur 2 ein Blockschaltbild eines digitalen Signalprozessors eines erfindungsgemäßen Hörgerätes,
Figur 3 ein Blockschaltbild eines Mittels zur Signalerzeugung des Signalprozessors des Hörgerätes, das eine Signalquelle und ein Mittel zur Signalveränderung umfaßt,
Figur 4 ein Blockschaltbild eines Mittels zur Signalerzeugung des Signalprozessors des Hörgerätes, das ein Mittel zur Signalveränderung umfaßt, dem externe Signale zur Tinnitus-Therapie zuführbar sind.

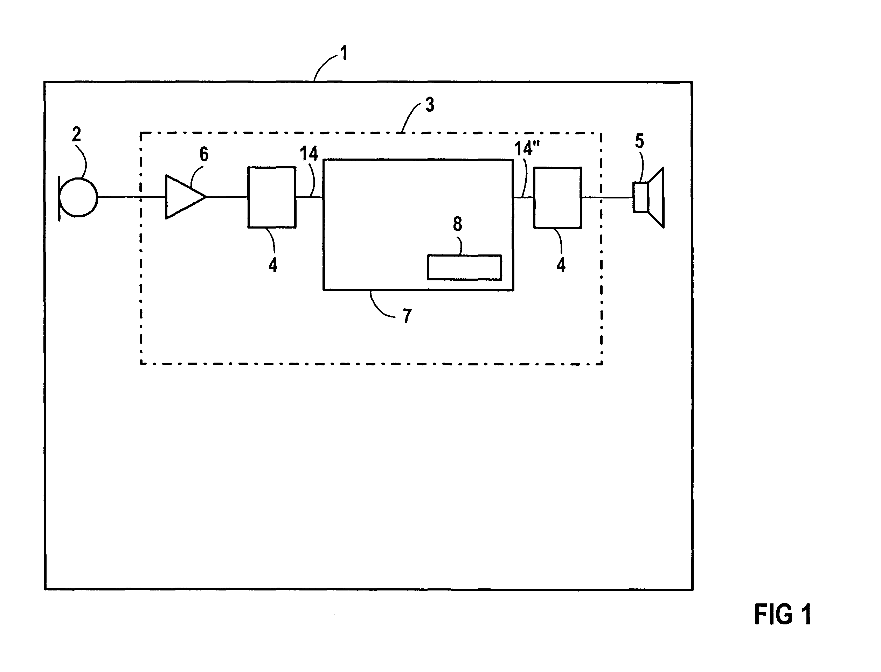

Gemäß Figur 1 besteht das Hörgerät 1 aus einem Eingangswandler 2, einer Signalverarbeitungseinrichtung 3 und einem Ausgangswandler 5. Die Signalverarbeitungseinrichtung 3 umfaßt insbesondere einen Verstärker 6, Signalwandler 4 und einen Signalprozessor 7. Der Signalprozessor 7 verarbeitet das digitalisierte Nutzsignal 14. Dazu steht dem Signalprozessor 7 ein Speicher 8 zur Verfügung.

Nach der Erfindung wird ein derartiges digitales Hörgerät zur Tinnitus-Therapie verwendet, wobei im Signalprozessor 7 beliebige Signale erzeugt und mit dem digitalisierten Nutzsignal 14 kombiniert werden können. Damit wird eine höhere Flexibilität als mit herkömmlichen analogen Hörgeräten zur Tinnitus-Therapie erreicht. Beispielsweise ist eine detaillierte Bestimmung des Tinnitus hinsichtlich des Frequenzgehaltes, des Pegels und/oder der Zeitstruktur und mit Hilfe dieser Ergebnisse eine individuelle Optimierung der Signale zur Tinnitus-Therapie für einen Patienten möglich. Darüber hinaus kann das Gerät jederzeit bei einer Veränderung des Tinnitus oder einer Veränderung der Therapie umprogrammiert werden, ohne daß der Patient ein neues Hörgerät benötigt oder Teile des Hörgerätes ausgetauscht werden müssen. Die Beschränkung der bisherigen Hörgeräte auf die Verwendung von Rauschsignalen entfällt, da z.B. auch melodische Klangfolgen oder sonstige Signale generierbar sind. Die Kombination eines Gerätes zur Tinnitus-Therapie mit einem digitalen Hörgerät hat den weiteren Vorteil, daß dieses Gerät auch für Normalhörende nutzbar ist. Die Normalhörenden können das digitale Hörgerät als Kommunikationsgerät verwenden, das z.B. bei Überempfindlichkeit gegenüber lauten Nutzsignalen diese in einen angenehmen Pegelbereich transformiert oder die im Nutzsignal vorhandenen Störgeräusche reduziert.

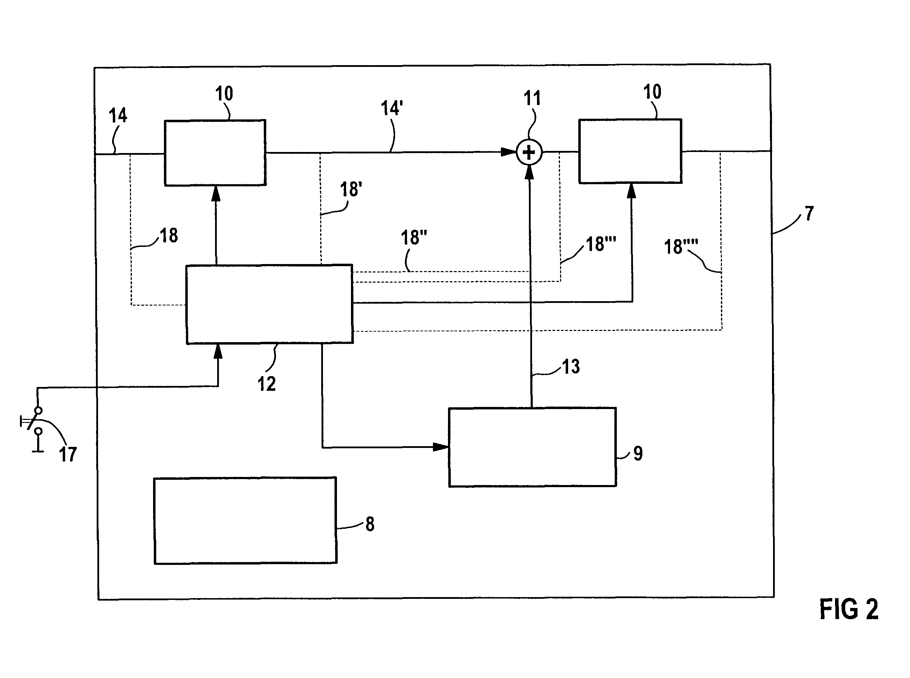

Figur 2 zeigt den Aufbau des Signalprozessors 7. Das digitalisierte Nutzsignal 14 wird durch Mittel 10 zur Signalbeeinflussung verarbeitet. Zusätzlich zu diesen Funktionen eines digitalen Hörgerätes werden mit Hilfe der Mittel 9 zur Signalerzeugung die Signale des Signalerzeugers 13 hergestellt und mit dem Mittel 11 mit dem digitalisierten Nutzsignal 14' kombiniert. Die Erfindung sieht vor, daß die Kombination nicht nur eine Addition, sondern auch eine Modulation od.dgl. sein kann. Der Signalprozessor 7 enthält darüber hinaus ein Mittel 12 zur Signalanalyse. Dieses Mittel 12 analysiert das digitalisierte Nutzsignal 14, 14', 14'' und evt. zusätzlich die Signale von Steuerelementen 17 und steuert damit die Mittel 9 zur Signalerzeugung und evt. zusätzlich die Mittel 10 zur Signalbeeinflussung. Das digitalisierte Nutzsignal wird dazu an verschiedenen Stellen 18, 18', 18'', 18''', 18'''' der Signalverarbeitungseinrichtung 3 abgegriffen. Dieser Mechanismus erlaubt beispielsweise eine Steuerung der Signalerzeugung 9 in Abhängigkeit vom digitalisierten Nutzsignal 14. Das digitale Nutzsignal kann hinsichtlich seiner Intensität, seiner spektralen Verteilung und/oder seiner Zeitstruktur ausgewertet werden und das Mittel 9 zur Signalerzeugung so steuern, daß ein gegenläufiges Verhalten erzielbar ist.

Der Vorteil einer solchen Steuerung ist z.B., daß eine ständige Stimulierung des Patienten möglich ist, ohne daß die Nutzsignale von den Signalen zur Tinnitus-Therapie überdeckt werden, da die Signale zur Tinnitus-Therapie nur dann eingeschaltet werden, wenn kein Nutzsignal vorliegt. Dabei sind beliebige Übergangszeiten zwischen Ende des Nutzsignals und Beginn der Signale zur Tinnitus-Therapie einstellbar.

Eine Variante dieser Ausführungsform ist die Analyse des Spektrums der digitalisierten Nutzsignale. Sind dabei in dem Frequenzbereich, der zur Therapie des Tinnitus notwendig ist, genügend hohe Signalpegel vorhanden, dann wird das Mittel 9 zur Signalerzeugung nicht aktiviert. Wird jedoch ein einstellbarer Schwellenwert unterschritten, dann werden die Signale 13 zur Tinnitus-Therapie mit dem digitalisierten Nutzsignal 14' kombiniert. Dadurch erreicht man eine ständige Stimulierung des entsprechenden Frequenzbereiches.

Nach einer Ausführung der Erfindung kann das Speichermittel 8 dazu verwendet werden, daß eine zeitabhängige Signalerzeugung und/oder Signalveränderung für die Tinnitus-Therapie programmierbar ist. Da Tinnitus oft zeitlich veränderlich ist, kann das Speichermittel so programmiert werden, daß sich die Signale zur Tinnitus-Therapie z.B. in Abhängigkeit von der Tageszeit verändern, so daß morgens andere Signale angeboten werden als abends, oder beim Einschlafen die Signale immer leiser werden und sich dann Nachts abschalten.

Gemäß einer weiteren Variante der Erfindung kann der Patient mit Hilfe der Steuerelemente 17 über das Mittel 12 zur Signalanalyse das Mittel 9 zur Signalerzeugung und/oder die Mittel 16 zur Signalveränderung hinsichtlich des Pegels, des Frequenzgehaltes und/oder der Zeitstruktur der Signale zur Tinnitus-Therapie beeinflussen. Dabei kann der Patient für jede Situation die Einstellung optimieren. Vorteilhaft ist auch die Auswahl von verschiedenen Programmen durch den Patienten, wobei diese Programme z.B. die Verwendung des Gerätes als Hörgerät ohne Signale zur Tinnitus-Therapie, als Gerät zur Erzeugung von Signalen zur Tinnitus-Therapie ohne Kombination mit dem Nutzsignal und/oder als Kombination von Hörgerät mit den Signalen zur Tinnitus-Therapie ermöglichen. Diese Auswahl ist vorteilhaft, da nicht immer die Signale zur Tinnitus-Therapie oder nicht immer das Nutzsignal benötigt wird, je nach Ausprägung des Tinnitus, je nach Therapieverfahren oder je nach Hörverlust des Patienten. Damit der Patient nicht immer das gleiche Signal zur Tinnitus-Therapie wahrnehmen muß, ist auch ein automatischer Wechsel oder ein manueller Wechsel mittels der Steuerelemente 17 zwischen unterschiedlichen natürlichen oder technisch erzeugten Signalen zur Tinnitus-Therapie möglich, welche im Speicher 8 gespeichert und/oder über die Signalquelle 15 abrufbar sind. Dadurch kannn ein Gewöhnungseffekt an die Signale vermieden oder vermindert werden und eine Lästigkeit wird zudem verringert.

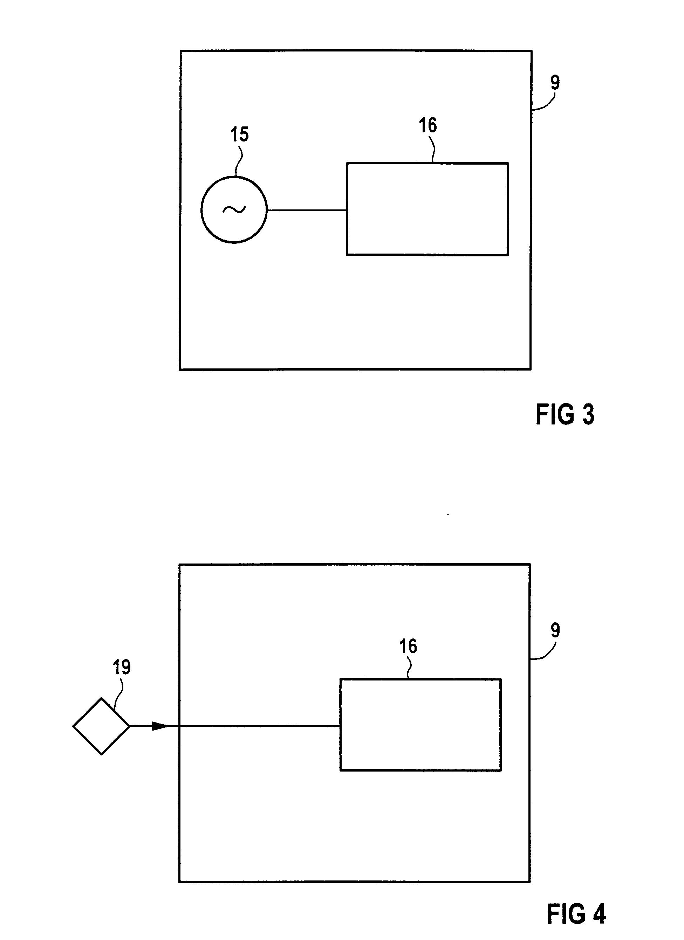

Figur 3 zeigt im Detail den Aufbau des Mittels 9 zur Signalerzeugung. Dieses Mittel 9 besteht aus einer Signalquelle 15 und einem Mittel 16 zur Signalveränderung. Die Signalquelle kann aus verschiedenen Ausführungsformen bestehen. Eine erste Ausführungsform ist ein rückgekoppeltes Schieberegister. Eine zweite Ausführungsform kann ein Speichermittel bilden, in welchem digitalisierte Signale abgelegt und auslesbar sind. Eine dritte Ausführungsform kann aus in Speichermitteln abgelegten Berechnungsvorschriften zur Generierung von Signalen bestehen. Die digitalisierten Signale, die die Signalquelle 15 verlassen, werden mit dem Mittel 16 zur Signalveränderung verarbeitet. Dabei können sie spektral oder zeitlich verformt oder im Pegel beeinflußt werden. Auch eine Kombination dieser Mittel kommt in Betracht. Damit können die Signale zur Tinnitus-Therapie für jeden Patienten individuell auf seine Bedürfnisse eingestellt werden. Die Limitierungen eines analogen Gerätes entfallen.

Figur 4 zeigt eine Variante zum Aufbau des Mittels 9 zur Signalerzeugung. Dieses Mittel umfaßt auch ein Mittel 16 zur Signalveränderung. Die Signale werden jedoch nicht in der Signalquelle 15 erzeugt, sondern gelangen durch den externen Signaleingang 19 in das Gerät. Der externe Signaleingang 19 kann z.B. ein Audioeingang des Hörgerätes oder ein Empfänger für AM- oder FM-Signale sein. Mit Hilfe dieses externen Signaleingangs 19 können außerhalb des Hörgerätes erzeugte künstliche oder natürliche Signale dem Patienten in beliebiger Verarbeitungsform zur Tinnitus-Therapie zur Verfügung gestellt werden.

## Patentansprüche

1. Programmierbares digitales Hörgerät (1) mit wenigstens einem elektroakustischen Eingangswandler (2), einer Signalverarbeitungseinrichtung (3), umfassend Signalwandler (4), Verstärker (6), einen digitalen Signalprozessor (7) und Speichermittel (8), und mit einem elektroakustischen Ausgangswandler (5), **dadurch gekennzeichnet, daß** das Hörgerät (1) als Gerät zur Tinnitustherapie mittels des Signalprozessors (7) Signale erzeugt, wobei der Signalprozessor (7) Mittel (16) zur Signalveränderung und als Mittel (9) zur Signalerzeugung eine Signalquelle (15) umfaßt, deren Signale (13) mit dem digitalisierten Audionutzsignal (14) kombinierbar sind, und wobei der Signalprozessor (7) Mittel (12) zur Signalanalyse umfaßt, die das digitalisierte Audionutzsignal (14) analysieren und in Abhängigkeit davon die Mittel (9) zur Signalerzeugung steuern.

2. Hörgerät nach Anspruch 1, **dadurch gekennzeichnet, daß** die Signalquelle (15) aus einem rückgekoppelten Schieberegister oder aus Speichermitteln, in denen digitalisierte Signale abgelegt und auslesbar sind, besteht.

3. Hörgerät nach Anspruch 1 oder 2, **dadurch gekennzeichnet, daß** zur Signalerzeugung in Speichermitteln abgelegte Berechnungsvorschriften vorgesehen sind.

4. Hörgerät nach einem der Ansprüche 1 bis 3, **dadurch gekennzeichnet, daß** als Mittel (16) zur Signalveränderung eine spektrale Formung, eine zeitliche Formung (Modulation), Mittel zur Pegelbeeinflussung oder eine Kombination dieser Mittel vorgesehen sind.

5. Hörgerät nach einem der Ansprüche 1 bis 4, **dadurch gekennzeichnet, daß** den Mitteln (12) zur Signalanalyse Signalabgriffe (18, 18', 18'', 18''', 18'''') zu verschiedenen Stellen der Signalverarbeitungseinrichtung (3) zugeordnet sind.

6. Hörgerät nach einem der Ansprüche 1 bis 5, **gekennzeichnet durch** einen externen Signaleingang (19) zur Einspeisung eines externen Signals zur Tinnitustherapie zum Mittel (16) zur Signalveränderung.

7. Hörgerät nach einem der Ansprüche 1 bis 6, **dadurch gekennzeichnet, daß** eine zeitabhängige Signalerzeugung und/oder Signalveränderung für die Tinnitustherapie über die Speichermittel (8) programmierbar ist.

8. Hörgerät nach einem der Ansprüche 1 bis 7, **dadurch gekennzeichnet, daß** als Mittel (11) zur Kombination der digitalisierten Audionutzsignale (14, 14') und der Signale (13) des Signalerzeugers ein Addierer, ein Modulator od.dgl. vorgesehen ist.

9. Hörgerät nach einem der Ansprüche 1 bis 8, **dadurch gekennzeichnet, daß** die Mittel (12) zur Signalanalyse so ausgebildet sind, daß durch Auswertung von Intensität, spektraler Verteilung und/oder Zeitstruktur des digitalisierten Audionutzsignales die Mittel (9) zur Signalerzeugung derart steuerbar sind, daß ein gegenläufiges Verhalten zwischen dem Audionutzsignal (14) und dem Signal (13) des Mittels (9) zur Signalerzeugung erzielbar ist.

10. Hörgerät nach einem der Ansprüche 1 bis 9, **dadurch gekennzeichnet, daß** in Speichermitteln des Hörgerätes oder im Speicher (8) des Signalprozessors (7) unterschiedliche natürliche oder technisch erzeugbare Signale zur Tinnitustherapie ablegbar und diese Signale vom Benutzer wahlweise abrufbar sind.

11. Hörgerät nach einem der Ansprüche 1 bis 10, **dadurch gekennzeichnet, daß** über die Signalquelle (15) unterschiedliche Signale zur Tinnitustherapie abrufbar sind.

## Claims

1. Programmable digital hearing aid (1) with at least one electroacoustic input transducer (2), a signal processing unit (3), comprising a signal converter (4), amplifier (6), a digital signal processor (7) and memory device (8), and with an electroacoustic output transducer (5), **characterised in that** the hearing aid (1) as a device for treating tinnitus generates signals by means of the signal processor (7), whereby the signal processor (7) has means (16) for signal changing and a signal source (15) as a means (9) of signal generation, whose signals (13) can be combined with the digitised wanted audio signal (14), and whereby the signal processor (7) has a means (12) for signal analysis, which analyses the digitised wanted audio signal (14) and controls the means (9) for signal generation in relation to this.

2. Hearing aid in accordance with claim 1, **characterised in that** the signal source (15) consists of a feedback shift register or memory devices in which digitised signals are stored and from which they can be read.

3. Hearing aid in accordance with claim 1 or 2, **characterised in that** calculation instructions stored in memory devices are provided for signal generation.

4. Hearing aid in accordance with one of claims 1 to 3, **characterised in that** a spectral shaping, a time modulation, means for influencing the level, or a combination of these means, are provided.

5. Hearing aid in accordance with one of claims 1 to 4, **characterised in that** as the means (12) for signal analysis signal taps (18, 18', 18", 18"', 18"") are assigned to various points of the signal processing device (3).

6. Hearing aid in accordance with one of claims 1 to 5, **characterised by** an external signal input (19) for supplying an external signal for treating tinnitus to the means (16) for signal changing.

7. Hearing aid in accordance with one of claims 1 to 6, **characterised in that** a time-dependent signal generation and/or signal changing for treating tinnitus can be programmed by means of the storage devices (8).

8. Hearing aid in accordance with one of claims 1 to 7, **characterised in that** an adder, a modulator or similar is provided as a means (11) of combining the digitised wanted audio signals (14, 14') and signals (13) of the signal generator.

9. Hearing device in accordance with one of claims 1 to 8, **characterised in that** the means (12) for signal analysis are formed such that by evaluating the intensity, spectral distribution and/or time structure of the digitised wanted audio signal, the means (9) for signal generation can be controlled in such a way that an opposing behaviour between the wanted audio signal (14) and the signal (13) of the means (9) for signal generation can be achieved.

10. Hearing aid in accordance with one of claims 1 to 9, **characterised in that** different natural or technically-generatable signals for treating tinnitus can be stored in the storage means of the hearing aid or in the storage device (8) of the signal processor (7) and that these signals can be called up as required by the user.

11. Hearing aid in accordance with one of claims 1 to 10, **characterised in that** different signals for treating tinnitus can be called up via the signal source (15).

## Revendications

1. Prothèse auditive numérique programmable (1) ayant au moins un convertisseur d'entrée électroacoustique (2), ayant un dispositif de traitement de signal (3), comprenant des convertisseurs de signal (4), des amplificateurs (6), un processeur de signal numérique (7) et des mémoires (8), et ayant un convertisseur de sortie électroacoustique (5), **caractérisée par** le fait que la prothèse auditive (1) en tant qu'appareil pour la thérapie pour acouphènes produit des signaux au moyen du processeur de signal (7), le processeur de signal (7) comprenant des moyens (16) destinés à la modification de signal et, comme moyens (9) destinés à la production de signal, une source de signal (15) dont les signaux (13) peuvent être combinés avec le signal audio utile numérisé (14), et le processeur de signal (7) comprenant des moyens (12) destinés à l'analyse de signal qui analysent le signal audio utile numérisé (14) et qui commandent en fonction de cette analyse les moyens (9) destinés à la production de signal.

2. Prothèse auditive selon la revendication 1, **caractérisée par** le fait que la source de signal (15) est constituée d'un registre à décalage couplé en rétroaction ou de mémoires dans lesquelles des signaux numérisés sont mémorisés et peuvent être lus.

3. Prothèse auditive selon la revendication 1 ou 2, **caractérisée par** le fait que des règles de calcul mémorisées dans des mémoires sont prévues. pour la production de signal.

4. Prothèse auditive selon l'une des revendications 1 à 3, **caractérisée par** le fait qu'il est prévu comme moyens (16) pour la modification de signal une mise en forme spectrale, une mise en forme temporelle (modulation), un moyen pour influencer le niveau ou une combinaison de ces moyens.

5. Prothèse auditive selon l'une des revendications 1 à 4, **caractérisée par** le fait que des prises de signal (18, 18', 18", 18"', 18"") sont associées aux moyens (12) destinés à l'analyse de signal en différents points du dispositif de traitement de signal (3).

6. Prothèse auditive selon l'une des revendications 1 à 5, **caractérisée par** une entrée de signal externe (19) pour l'introduction d'un signal externe destiné à la thérapie pour acouphènes jusqu'aux moyens (16) destinés à la modification de signal.

7. Prothèse auditive selon l'une des revendications 1 à 6, **caractérisée par** le fait qu'une production de signal en fonction du temps et/ou une modification de signal en fonction du temps en vue de la thérapie pour acouphènes peuvent être programmées par l'intermédiaire des mémoires (8).

8. Prothèse auditive selon l'une des revendications 1 à 7, **caractérisée par** le fait qu'il est prévu comme moyen (11) pour la combinaison des signaux audio utiles numérisés (14, 14') et des signaux (13) du générateur de signal un additionneur, un modulateur ou un opérateur analogue.

9. Prothèse auditive selon l'une des revendications 1 à 8, **caractérisée par** le fait que les moyens (12) destinés à l'analyse de signal sont conçus de telle sorte que, par l'évaluation de l'intensité, de la distribution spectrale et/ou de la structure dans le temps du signal audio utile numérisé, les moyens (9) destinés à la production de signal peuvent être commandés de manière à pouvoir obtenir un comportement opposé entre le signal audio utile (14) et le signal (13) du moyen (9) destiné à la production de signal.

10. Prothèse auditive selon l'une des revendications 1 à 9, **caractérisée par** le fait que, dans des mémoires de la prothèse auditive ou dans la mémoire (8) du processeur de signal (7), il peut être mémorisé différents signaux, naturels ou pouvant être produits techniquement, destinés à la thérapie pour acouphènes et que ces signaux peuvent être appelés à volonté par l'utilisateur.

11. Prothèse auditive selon l'une des revendications 1 à 10, **caractérisée par** le fait que différents signaux destinés à la thérapie pour acouphènes peuvent être appelés par l'intermédiaire de la source de signal (15).
